# EUROPÄISCHE PATENTSCHRIFT

(11) **EP 0 950 423 B1**
(45) Veröffentlichungstag und Bekanntmachung des Hinweises auf die Patenterteilung: **13.10.2004**
(21) Anmeldenummer: 99106573.1
(22) Anmeldetag: 31.03.1999
(51) Int. Cl.: A61M 15/00, B65D 83/00, B65D 85/00

(54) **Spender für Medien, insbesondere Pulver**
Product dispenser, especially for powder
Distributeur de produit, en particulier pour une poudre

(30) Priorität: 18.04.1998 DE 19817417
(43) Veröffentlichungstag der Anmeldung: 20.10.1999
(73) Patentinhaber: Ing. Erich Pfeiffer GmbH, 78315 Radolfzell (DE)
(72) Erfinder: Fuchs, Karl-Heinz, 78315 Radolfzell (DE)
(74) Vertreter: Patentanwälte Ruff, Wilhelm, Beier, Dauster & Partner

(56) Entgegenhaltungen:
- WO-A-96/09085
- WO-A-97/09082
- WO-A-98/34660
- DE-A- 4 021 263
- US-A- 5 533 502
- US-A- 5 709 202

## Beschreibung

Die Erfindung betrifft einen Spender für Medien. Diese können wenigstens teilweise fest bzw. fließfähig, wie flüssig, pastös und/oder gasförmig sein. Bevorzugt sind sie rieselfähig bzw. pulverförmig und daher als Schüttgut geeignet. Der Spender soll einhändig gehalten und gleichzeitig mit den Fingern der haltenden Hand betätigt werden können, z.B. um pharmazeutische, kosmetische oder ähnliche Medien auszubringen. Solche Medien können zur Applizierung in den Atemwegen bestimmt und nasal oder oral einzubringen sein.

Der Spender kann nur für einen einzigen Austragvorgang bzw. eine einzige, gleichgerichtete Hubbewegung vorgesehen sein. Dann sind Rückstellmittel zur Rückstellung aus der Endstellung in die Ausgangs- oder Ruhestellung nicht erforderlich.

Zum Austrag kleiner, dosierter Medienmengen von höchstens 30, 20, 15 oder 12 und mindestens 5 mg genügen sehr kompakte Spender. Die Speicherkammer enthält das Medium zweckmäßig entsprechend einer auszutragenden Einzeldosis vorportioniert und kann langgestreckt kapsel- oder napfförmig sein. Vorteilhaft ist sie flachschalen- und/oder kugelkalottenförmig. So kann das Medium mit einem Zweit- oder Förderfluid, wie einer Flüssigkeit, Gas oder Luft, aus der geöffneten Speicherkammer ausgeschwemmt werden. Das Medium wird dabei im Förderfluid zerstäubt. So wird eine möglichst gleichmäßige Partikelverteilung und -vereinzelung bis zum Austritt aus dem Medienauslaß erzielt.

Die Napftiefe der Speicherkammer kann unter 7, 5 oder 4 und über 2 mm liegen. Der größte Durchmesser der Kammer liegt unter 20, 15 und 10 mm und über 3 oder 6 mm, so daß er größer als die Napftiefe ist. Der Speicher ist vorteilhaft auswechselbar und nach Entleerung erneuerbar. Das Kammervolumen des Speichers ist nur teilweise mit Medium gefüllt. So wird ab Anbeginn des Austrages die Auflockerung erleichtert. Der Speicher- oder Magazinkörper kann formstabil sein. Er ist jedoch zweckmäßig im Anschluß an die formstabile Speicherkammer biegeflexibel, nämlich ähnlich einem durchsichtigen oder undurchsichtigen bzw. nicht transluzenten Blister durch foliendünne Wandungen gebildet. Vor dem Austrag ist die gefüllte Speicherkammer druckdicht und steril verschlossen. Der Verschluß ist ebenfalls durch eine Folie aus Kunststoff oder Metall, wie Aluminium, gebildet. Sie ist haftend bzw. durch Versiegelung befestigt und überdeckt die Kammeröffnung eben. Die Folie schließt bis unmittelbar an die Begrenzung der Kammeröffnung sowie über deren Umfang durchgehend dicht an den Kammerkörper an. So kann aus der Kammer kein Medium zwischen Kammerkörper und Verschluß eindringen siehe z.B. WO 98/34660, EP-A 844 007 oder DE-OS 195 02 725.

Die DE 4021263 A1 zeigt einen Spender, gemäß dem Oberbegriff des Anspruchs 1, der in einem drehbaren Kranz zahlreiche ampullenartige Behälter für flüssige Medien aufnimmt, die mittels eines Stopfens mit einer durchstoßbare Haut verschlossen sind. Diese wird bei Benutzung durch Zusammendrücken zweier Gehäuseteile durchstoßen und der Inhalt des jeweils in Benutzungsposition stehenden Behälters durch eine Zerstäuberdüse ausgetragen. Dabei wirkt der Stopfen als Kolben einer Schubkolbenpumpe.

Die US 5533502 A betrifft einen Spender, der als Speicher einen massiven Ring mit Kammern, eine entsprechende durchbrochene Leiste oder einen mit Verzahnungen versehenen Ringkranz aufweist.

Die WO 96/09085 beschreibt ein sehr aufwendiges Verabreichungsgerät für pulverförmige Medikamente, das mit einem flexiblen Blisterstreifen arbeitet.

Der Erfindung liegt die Aufgabe zugrunde, einen Spender für Medien zu schaffen, bei welchem Nachteile bekannter Ausbildungen vermieden bzw. Vorteile von genannten Ausbildungen gewährleistet sind. Insbesondere soll eine Überführung des Speichers in unterschiedliche Funktionsstellungen ermöglicht sein, z.B. um aufeinanderfolgend unterschiedliche Funktionen des Spenders auszulösen.

Diese Aufgabe wird durch die Merkmale des Anspruchs 1 gelöst.

Es sind Mittel vorgesehen, welche betriebsbedingt bzw. im Laufe eines Austragzyklus' oder einer zugehörigen Betätigung eine Funktionsbewegung des Speichers bewirken. Eine solche Bewegung kann eine lineare Hubbewegung oder eine quer dazu liegende Schaltbewegung oder eine zur Schaltbewegung querliegende Öffnungsbewegung sein. Durch die Schaltbewegung wird die Speicherkammer aus einer geschützten Ruhelage in die Austragsposition bewegt. Durch die Öffnungsbewegung wird eine Wandung, insbesondere der als Auslaßverschluß vorgesehene Kammerdeckel, mit einer Auslaßöffnung für das Medium versehen. Durch die Hubbewegung kann der gesamte Spender so verkürzt werden, daß er während des Austrages besonders handlich ist. Vorteilhaft beginnt die Öffnungsbewegung kurz vor oder mit der Schaltbewegung. Letztere endet entweder bevor das Öffnungsglied in Eingriff mit dem Verschluß der Speicherkammer kommt oder danach, jedoch bevor die Öffnungsbewegung beendet ist. Im letzten Fall führt das Öffnungsglied dann, während es schon in Eingriff mit dem Klammerverschluß steht, zwei zueinander querliegende Bewegungen aus. Dadurch wird der Verschluß besonders wirkungsvoll bzw. auf großer Weite geöffnet. Die Hubbewegung kann dazu dienen, einen weiteren Auslaßverschluß, wie ein Ventil, zu steuern bzw. zu öffnen, ein Fördermedium zu fördern, den Arbeitshub durch Anschlag zu begrenzen, Fördermedium anzusaugen o.dgl.. Ein Speicherträger nimmt den Speicher auswechselbar auf und ist vorteilhaft synchron mit dem Speicher bewegbar, insbesondere über den Funktionshub bzw. die Schaltbewegung, nicht jedoch über den Arbeitshub. Der Speicherträger bildet einen Begrenzer, wie einen Zylinder oder Kolben einer Druck- oder Pumpkammer. Der Träger umfaßt die Öffnungsmittel für den Auslaßverschluß und bildet den Anschlag. Der Träger verengt vor Strömungsbeginn des Fördermediums dessen Auslaßkanal, der zur Speicherkammer führt. Ferner kann der Speicherträger eine Zähleinrichtung für Austragzyklen bzw. ein Anzeigeglied dieser Zähleinrichtung bilden, die gegenseitige Lagerung der beiden Gehäuse-Einheiten des Spenders verbessern bzw. versteifen, eine an eine äußerste Wandung des Spenders anschließende Ringkammer begrenzen, ein Widerlager für eine Rückstellfeder oder für einen Speicherniederhalter bilden, den Speicher bewegbar bzw. drehbar, jedoch zentriert, aufnehmen, eine zum Arbeitshub quer federnde Verspannung gegenüber einer der Einheiten bewirken usw.

Im Betrieb ist der Speicher bzw. Speicherträger zweckmmäßig vollständig umschlossen innerhalb eines Gehäuses vorgesehen. Dessen Gehäuseteile sind unmittelbar durch die beiden Einheiten gebildet. Zum Auswechseln des Speichers bzw. für dessen manuelle Zugänglichkeit ist das Gehäuse zu öffnen. Hierzu könnten zwar die beiden Gehäuseteile vollständig voneinander getrennt werden. Zweckmäßig jedoch weist einer der Gehäuseteile eine Zugangsöffnung auf. Sie ist im Betrieb mit einem Deckel, wie einem Stirndeckel, verschlossen. Dieser Deckel kann einen Auslaßstutzen mit dem Medienauslaß aufweisen. Der Deckel kann auch folgendes umfassen: das Öffnungsglied, ein Schaltglied des Stelltriebes, Sicherungsmittel zur spielfreien Lagesicherung des Speichers, eine Betätigungshandhabe, ein Widerlager für eine Feder o.dgl.. Der Deckel kann axial aufsetzbar, radial einschiebbar oder schwenkbar am zugehörigen Gehäuseteil gelagert sein. Der Deckel begrenzt denjenigen Teil des Auslaßkanales für das Fördermedium, der zur Speicherkammer führt. Er begrenzt auch denjenigen Auslaßkanal, der von der Speicherkammer zum Medienauslaß führt. Alle diese Funktionen sind durch einen einteiligen oder mehrteiligen Bauteil gebildet. Der zugehörige, übrige Gehäuseteil zentriert den Speicher bzw. Speicherträger und bildet eine Abzugsicherung. Sie sichert die zugehörige Einheit gegen axiales Abziehen von der anderen Einheit. Außerdem kann dieser Gehäuseteil den Speicher bzw. den Speicherträger über den zugehörigen Hubweg gegenüber dem anderem Gehäuseteil mitnehmen. In der Schließstellung sind der Deckel und der dazugehörige Gehäuseteil über eine Schnappverbindung gegeneinander gesichert, die als Dichtung wirkt.

Die Schaltbewegung kann bewirkt werden durch axial in Eingriff miteinander gelangende Umfangszahnungen mit schrägen Flanken, die ähnlich wie bei einer Zähleinrichtung ausgebildet sind, siehe z.B. EP-B 114 617.

Ausführungsbeispiele der Erfindung sind in den Zeichnungen dargestellt und werden im folgenden näher erläutert. In den Zeichnungen zeigen:
- Fig. 1: einen erfindungsgemäßen Spender in Ausgangsund geöffneter Stellung während des Magazinwechsels, teilweise im Axialschnitt,
- Fig. 2: den Spender gemäß Fig. 1 in geschlossenem Zustand und vergrößerter Darstellung,

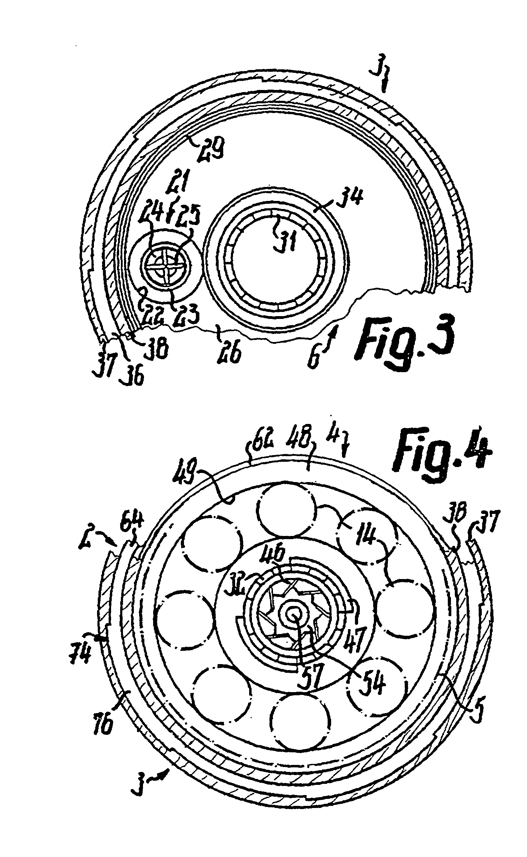

- Fig. 3: einen nach oben gerichteten Querschnitt durch den Spender und
- Fig. 4: einen nach unten gerichteten Querschnitt durch den Spender.

Der Spender 1 weist zwei manuell gegeneinander axial bewegbare Basis-Einheiten 2, 3 auf. Sie bilden die voneinander abgekehrten Enden des Spenders 1 und sind unter dessen Verkürzung zusammendrückbar. Die Einheiten 2, 3 bilden ein Gehäuse, in dem eine weitere Einheit 4 vollständig liegt. Deren Außendurchmesser ist etwa gleich dem größten Innendurchmesser der ersten Einheit 2. Die Länge der Einheit 4 entspricht mindestens einem Drittel oder der Hälfte des Abstandes zwischen den großen äußeren Stirnflächen bzw. den Handhaben der Einheiten 2, 3. Eine weitere Einheit 5, nämlich ein Speicher, ist vollständig innerhalb des Gehäuses und zwischen einander zugekehrten Stirnflächen der Einheiten 3, 4 angeordnet. Die Einheit 5 hat gegenüber jeder der Einheiten 2 bis 4 eine wesentlich geringere Axialerstreckung. Die Einheiten 4, 5 sind gegeneinander, gemeinsam sowie unabhängig voneinander gegenüber jeder der Einheiten 2, 3 bewegbar, nämlich sowohl axial als auch in einer Quer- bzw. Drehbewegung. Die Einheiten 2, 3 sind permanent gegeneinander verdrehgesichert. Relativ zu jeder der Einheiten 2 bis 5 sowie gemeinsam mit der Einheit 3 ist auch eine weitere Einheit 6 bewegbar. Sie bildet eine Untereinheit der Einheit 3, nämlich einen abzieh- bzw. klappbaren Deckel des Gehäuses.

Gemeinsam mit jeder der Einheiten 3, 6 ist der Medienauslaß 7 relativ zu den Einheiten 2, 4, 5 bewegbar. Vollständig innerhalb des Gehäuses 2, 3 sind Mittel 8 zur Förderung eines Fördermediums, wie Luft, vorgesehen. Dieses Gas wird über einen Auslaß-Verschluß 9, wie ein federnd geschlossenes Ventil, zuerst dem Medium und dann dem Auslaß 7 zugeführt, der unmittelbar ins Freie mündet.

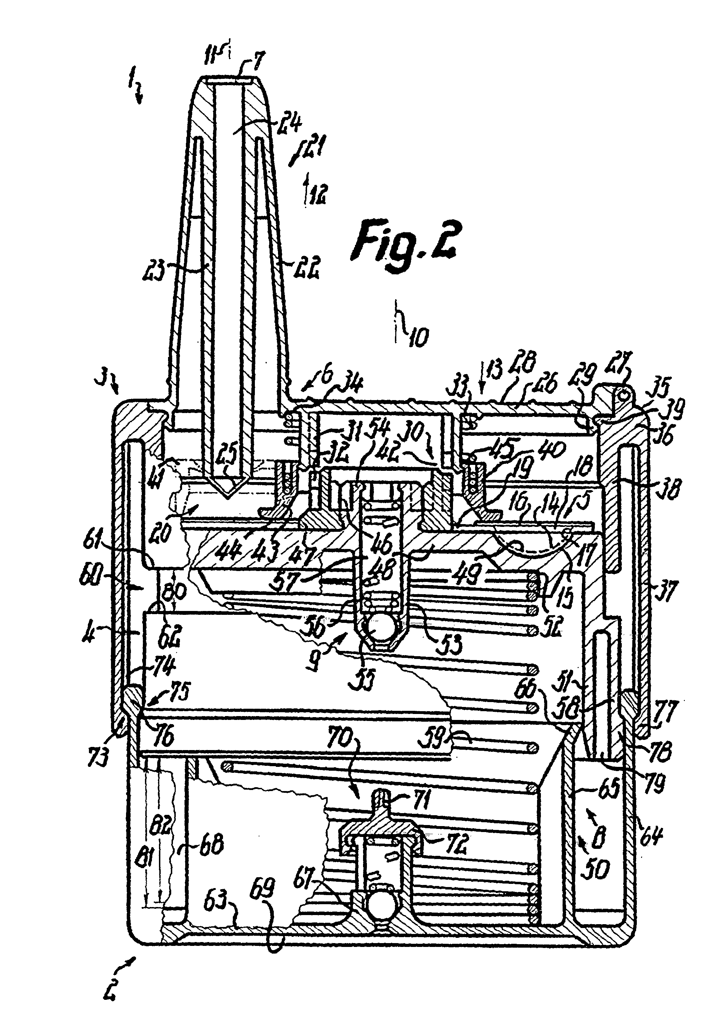

Die Achse 11 des Auslasses 7 liegt exzentrisch, und achsparallel zur Mittel- bzw. Hauptachse 10 der Einheiten 2 bis 5. Die Achse 11 liegt nahe benachbart zu den äußersten Umfängen der Einheiten 2 bis 5. Innerhalb der Einheiten 2, 4 und 5 sowie stromaufwärts im Anschluß an den Auslaß 7 strömt das Medium bzw. das mit diesem geförderte und zu applizierende Wirkmedium parallel zur Achse 10, 11 in Richtung 12 von der Einheit 2 weg. So verläßt es am Auslaß 7 den Spender 1 in Richtung 12. Aus der Ausgangsstellung gemäß Figur 2 werden die Einheiten 3 bis 6 relativ zur Einheit 2 in entgegengesetzter Achsrichtung 13 bei der Betätigung manuell bewegt.

Der Speicher 5 weist einen einteiligen, tiefgezogenen oder durch Spritzguß hergestellten Grundkörper auf. Der hat eine Mehrzahl von hier acht gleichmäßig um Achse 10 verteilten Kammerkörpern 15 und einen gesonderten, ebenen Auslaß- bzw. Folienverschluß 16. Jeder Körper 15 bildet eine kugelkalottenförmige Speicherkammer 14 mit einer im Verhältnis zum Kugelradius kleineren, maximalen Kammertiefe. Daher hat er eine bis zur ebenen Kammeröffnung 17 degressiv erweiterte Kammerbegrenzung. Die kugelkalottenförmigen Körper 15 stehen nur über eine Seite einer Flansch- oder Speicherplatte 18 vor, die einteilig mit den Körpern 15 ist. Die Körper 15 haben im Verhältnis zu ihrer Öffnungsweite kleinere Zwischenabstände und weisen die Öffnung 17 an der Seite der Platte 18 auf, die vom Körper 15 abgekehrt ist. Diese Seite wird auch von dem ringscheibförmigen Verschluß 16 abgedeckt. Der Innenumfang der Platte 18 ist mit mindestens einem Mitnehmer 19, beispielsweise einem Vorsprung, einer Zahnung o.dgl. versehen. Dieser Innenumfang greift axial bewegbar und abziehbar, jedoch in Drehrichtung formschlüssig in die Einheit 4 ein und nimmt bei der Drehbewegung der Einheit 4 um Achse 10 den Speicher 5 mit.

In der Achse 11, innerhalb des Gehäuses 2, 3 und zwischen den Einheiten 3, 4 sind Mittel 20 zum Öffnen des Verschlusses 16 vorgesehen. Der wird im Bereich jeweils einer einzelnen Öffnung 17 durch Zerstörung geöffnet. Die Mittel 20 gehören zu einem in der Achse 11 frei vorstehenden Austragstutzen 21. Der ist an seiner freien Endfläche von der Auslaßöffnung 7 durchsetzt. Der Stutzen 21 ist gerade und weist einen äußersten Mantel 22 auf, welcher zum Auslaß 7 spitzwinklig konisch verjüngt ist. Mit radialem Abstand innerhalb des Mantels 22 liegt ein innerer Mantel 23. Er ist über seine gesamte Länge bis zur Öffnung 7 von einem Auslaßkanal 24 durchsetzt. Dessen Weite ist mindestens 2 und höchstens 5 mm, wobei er durchgehend konstante Querschnitte hat. Die unterschiedlich langen Mäntel 22, 23 gehen am äußeren Ende des Stutzens 21 einteilig ineinander über. Der Mantel 23 steht in Richtung 13 über den Mantel 22 sowie in die Einheit 3 vor. Am inneren Ende ist der Mantel 23 einteilig mit einem Öffnungsglied 25, wie einem zugespitzten Stech- oder Reißglied, versehen. Mit dem wird durch eine Axial- bzw. Drehbewegung um die Achse 10 der Verschluß 16 jeweils an derjenigen Stelle gesprengt, an welcher eine Kammer 14 in der Achse 11 liegt. Der zentrale Radialabstand jeder Kammer 14 von der Mittelachse 10 des Speichers 5 ist gleich dem Abstand der Achsen 10, 11. Der Speicher kann auch entsprechend der EP-PS 591 182 mit Pumpkolben für das Medium arbeiten.

Die Einheit 6 ist an der Einheit 3 mit einem Lager 27 gelagert und über einen Winkel von genau 90° oder mehr schwenkbar. Die Lagerachse liegt rechtwinklig quer zur gemeinsamen Axialebene der Achsen 10, 11 sowie zwischen den Außen- und Innenumfängen der Einheit 3. Der Mantel 22 schließt mit seinem erweiterten Ende einteilig an eine ebene Stirnwand 26 an. Sie bildet die von der Einheit 2 entfernte Endwand der Einheit 3. An ihrer Außenseite bildet die Wand 26 die Handhabe 28 zur Druckbetätigung der Einheit 3, die mit Griffigkeits-Vorsprüngen versehen ist. Auf der Seite der Achse 10, die von der Achse 11 abgekehrt ist, bildet die Wand 26 den zugehörigen Lagerkörper des Lagers 27. Dessen Achse liegt nahe bei der Ebene der Handhabe 28. An ihrer Innenseite weist die Wand 26 ein Schnappglied 29 auf, das ringförmig entlang ihres Außenumfanges vorsteht, radial federt und in Betriebslage die Einheit 6 spielfrei an der Einheit 3 sichert.

In der Einheit 3 sind Sicherungs- bzw. Stellmittel 30 vorgesehen, die zwischen der Wand 6 und der Einheit 4 liegen. Die Mittel 30 schalten den Speicher 5 mit oder ohne Einheit 4 je Betätigungshub so um die Achse 10 weiter, daß die nächste, noch nicht geöffnete Kammer 14 in die Achse 11 kommt. Von der Innenseite der Wand 26 steht in Richtung 13 einteilig ein hülsenförmiges Stellglied 31 vor. Es liegt unmittelbar benachbart zum Mantel 22, ist mit der Wand 26 einteilig und hat gleichmäßig über seinen Innenumfang verteilte Drehglieder, nämlich Axialstege mit seitlich abgeschrägten Enden. Entgegen Richtung 13 stehen komplementäre Stellglieder 32 vor, nämlich am Außenumfang einer Hülse der Einheit 4. Die Glieder 32 können einteilig mit der Einheit 4 ausgebildet oder durch eine gesonderte Muffe gebildet sein. Ein Axialhub zwischen den Einheiten 3, 4 bewirkt durch die Glieder 31, 32, die dann erst in Eingriff miteinander gelangen, einen der genannten Schaltschritte des Speichers 5. Die Glieder 31, 32 sind eng von einer stets vorgespannten Druck-Feder 33 umgeben. Ihr eines Ende ist an der Innenseite der Wand 26 in einem Widerlager 34 abgestützt. Ihr anderes Ende ist am Speicher 5 bzw. dessen Teilen 16, 18 ebenfalls ringförmig abgestützt. Daher drückt die Feder 33 den Speicher 5 an die Einheit 4. Am Ende des anschlagbegrenzten Hubes des Stellmittels 30 liegt das Glied 32 mit geringem Abstand von der Wand 26.

Die Einheit 3 bildet einen Gehäuseteil 35 des Gehäuses. Der Teil 35 hat eine ringförmige Stirnwand 36, die an die Wand 26 anschließt. Von der Wand 36 steht nur in Richtung 13 ein äußerster Mantel 37 sowie ein innerer, kürzerer Mantel 38 frei vor. Der Mantel 38 hat Radialabstand vom Mantel 37. Von der Innenseite der Wand 36 geht daher eine schmale Ringnut aus, die von den Mänteln 37, 38 begrenzt ist. In diese Nut greift die Einheit 2 ein. Die Nut erstreckt sich nur über einen Teil der Länge des Mantels 37 und über die gesamte Länge des Mantels 38. An der Außenseite der Wand 36 liegt das Lager 27. Die Wand 36 weist eine Vertiefung auf, welche die Platte 26 vollständig versenkt aufnimmt. Die Platte 26 umgibt auch den Mantel 22. So schließen die Außenseiten der Wände 26, 27 bündig bzw. ebenengleich und lückenfrei aneinander an. Über den Innenumfang der Wand 36 und im Anschluß an diese Vertiefung steht ein Gegenglied 39 für das Schnappglied 29 vor. Der ineinander greifende Sitz der Glieder 26, 36 und 29, 39 ist druckdicht und wirkt auch als Labyrinthdichtung.

Zur axialen Lagesicherung des Speichers 5 an der Einheit 4 ist ein Sicherungskörper bzw. Niederhalter 40 vorgesehen. An ihm ist das zugehörige Ende der Feder 33 unmittelbar zentriert abgestützt. Statt der in durchgehenden Linien gezeigten Ausbildung kann der Körper 40 auch zusätzlich die strichpunktiert dargestellte Ausbildung 41 haben. Dann weist der Körper sowohl in der Achse 10 als auch in der Achse 11 einen Muffenteil 40 bzw. 41 auf. Für die Luft, die durch den Verschluß 9 zwischen die Einheit 4 und die Wand 26 strömt, bilden die ineinander greifenden Glieder 26, 31, 32, 40 eine zweifache Kanal- bzw. Querumlenkung 42. Zuerst ist eine Umlenkung quer zur Achse 10 und dann eine Umlenkung zurück in Richtung 13 zwischen den Umfängen der Glieder 31, 32 vorgesehen. Durch entsprechend reduzierte Durchlaßquerschnitte innerhalb dieser Rückumlenkung wird die Strömungsgeschwindigkeit stark erhöht. Die gesamte Luft wird nach der Rückumlenkung gezielt gegen den Boden der Kammer 14 gerichtet, die in der Achse 11 steht. Die Luft ist dabei von dem näher bei der Achse 10 liegenden Kammerbereich radial nach außen gerichtet.

Dabei kann die Strömung in der Kammer 14 und um die Achse 10 bzw. den Mantel 23 eine Wirbel- oder Rotationsströmung ausführen. Sie verläßt die Kammer 14 als Wendelströmung unmittelbar in den Kanal 24. Hierzu weist der Halter 40 als Druck- und Dichtfläche 44 und um die Achse 10 eine ringförmige Stirnfläche 44 auf. Sie liegt beim Drehen unter Vorspannung an der Außenseite des Verschlusses 16 feststehend oder gleitend an. Die Fläche 44 ist von einem nutförmigen, Querkanal 43 durchsetzt, der gegen die Achse 11 gerichtet ist. Die Luft durchströmt den Kanal 43 nach der Rückumlenkung so, daß sie schräg in den Boden der genannten Kammer 14 gerichtet ist. Das andere Ende des Halters 40 bzw. dessen Innenumfang greift mit einer Schnapp- und Dichtverbindung 45 in den Außenumfang des Gliedes 31 axial verschiebbar und entweder drehbar oder gegen Drehung gesichert ein. Am Ende des Hubes der Stellmittel 30 kann der Halter 40 an der Innenseite der Wand 26 bzw. am Lager 34 anschlagen. Dadurch wird dann seine Halte- bzw. Anpreßkraft mehrmals erhöht. Der Halter 40, 41 kann auch einteilig mit der Feder 33, der Wand 26 bzw. dem Glied 31 ausgebildet sein, z.B. als Balg.

Der Teil 41 ist zweckmäßig in Axialansicht langrund. Er schließt mit Abstand zwischen den Achsen 10, 11 einteilig an den Außenumfang des Teiles 40 an. An ihrem vom Speicher 5 entfernten Ende weist der Teil 41 eine Stirnwand auf, die vom Rohr 23 permanent durchsetzt ist und mit einer Dichtlippe am Außenumfang des Rohres 23 abgedichtet gleitet. So nimmt mit erhöhtem Fluiddruck innerhalb des Teiles 41 die Dichtpressung zu. Auch der Teil 41 bzw. dessen gesamter Umfang liegt mit einer Druckfläche 44 dicht am Verschluß 16 bzw. der Platte 18 an. Die Druckfläche 44 des Halters 40 übergreift die Öffnung 17 zu deren Mittelachse hin. Auch durch den Teil 41 bzw. verhältnismäßig enge Strömungsquerschnitte wird die Luft nur gegen die jeweils zu entleerende Kammer 14 gezielt gerichtet. Die übrigen Kammern 14 bzw. deren Verschlüsse 16 werden dabei nicht angeströmt.

Das gesonderte Hülsenglied 32 ist relativ zum Grundkörper der Einheit 4 um die Achse 10 drehbar und kann mit dem Grundkörper über eine Dreh- bzw. Freilaufkupplung 46 verbunden sein. Sie erlaubt eine Drehbewegung des Kupplungsgliedes 32 gegenüber dem Grundkörper in Drehrichtung der Schaltbewegung und sperrt in entgegengesetzter Drehrichtung formschlüssig. Das radial innere Kupplungsglied des Grundkörpers kann als Stern ausgebildet sein, der schräg abstehende und biegefedernde Sperrarme gemäß Figur 4 hat. An seinem Außenumfang weist das Kupplungsglied 32 einen Drehmitnehmer 47 auf, der zum Mitnehmer 19 komplementär ist. Der Teil 47 umfaßt zwei gleichmäßig über dem Umfang verteilte, radial vorstehende Nocken. Zwischen deren Seitenflanken greifen die Nocken des Mitnehmers 19 um die Achse 10 radial spielfrei ein. Diese Nocken und die Nocken 47 sind in Umfangsrichtung gleich groß.

Der einteilige Grundkörper der Einheit 4 weist eine ringförmige Stirnwand 48 auf. Von ihr steht in Richtung 13 ein Zylindermantel 51 ab. Beide Wände 48, 51 liegen radial innerhalb des Mantels 38 und schließen axial verschiebbar und abgedichtet an dessen Innenumfang an. An der Außenseite weist die Wand 48 für die Körper 15 eine vertiefte Aufnahme 49 auf. Sie ist durch über den Umfang verteilte Zentrier- bzw. Einzelvertiefungen oder durch eine um die Achse 10 durchgehende Ringnut gebildet, in welcher der Speicher 5 drehbar ist. An der Vertiefung 49 ist jeder Körper 15 wenigstens mit einem Großkreis bis zur Platte 18 durchgehend abgestützt. Die Platte 18 liegt dann bis zu den Mitnehmern 19 und um die bzw. beiderseits der Vertiefung 49 an der äußeren Stirnfläche der Wand 48 vollflächig an. An dieser Außenseite ist auch das Glied 32 axial abgestützt. Die Innenseite der Wand 48 bildet ein Widerlager 52 für eine Rückstell- bzw. Druckfeder 59, die relativ zur Feder 33 wesentlich stärker und stets stärker vorgespannt ist. Die Feder 59 greift radial zentriert in das Lager 52 ein. Ein rohrförmiges Gehäuse 53 des Verschlusses 9 steht radial innerhalb des Lagers 52 und in Richtung 13 über die Innenseite der Stirnwand 48 vor. Über die Außenseite der Wand 48 steht ein hohler Vorsprung 54 oder ein Zapfen der Kupplung 46 vor. Der sternförmige Außenumfang des Zapfens 54 ist mit den federnden Sperrgliedern der Kupplung 46 versehen. Diese greifen in den Innenumfang des Gliedes 32 ein. Das kann einteilig mit dem Grundkörper 4 ausgebildet sein, oder es ist axial auf den Zapfen 54 aufgesetzt und mit einer axialen Schnappsicherung spielfrei gesichert. Diese Sicherung kann gleichzeitig ein Drehlager zur Verdrehung des Gliedes 32 relativ zum Körper 4 sein.

Im freien Ende des Gehäuses 53 ist ein bewegbarer Verschluß- und Ventilkörper 55 oder eine Kugel angeordnet und mit einer Feder 56 gegen einen Ventilsitz des Gehäuses 53 belastet. Der Gehäuseraum der Hülsen 53, 54 nimmt die Teile 55, 56 auf und bildet einen Kanal, der wie die Hülsen 53, 54 in der Achse 10 liegt und die Mittel 8 mit dem Kanal 42 verbindet. Im Abstand von der Stirnwand 48 und mit Radialabstand ist der Mantel 51 von einem Außenmantel 58 umgeben. Der liegt ebenfalls permanent an der Einheit 2 an. Innerhalb der Mäntel ist die Feder 59 angeordnet.

Die einander zugekehrten Enden der Mäntel 38, 58 bilden Mitnahmemittel 60. Das freie Ende des Mantels 38 bildet einen Mitnehmer 61. Die Schulter am gegenüberliegenden Ende des Mantels 58 bildet den zugehörigen Anschlag. Der Innenumfang des Mantels 38 ist druckdicht am Außenumfang des Körpers 4 geführt. In Ausgangsstellung liegen die Flächen 61, 62 unter der Spannung der Feder 33 in demjenigen Abstand voneinander, welcher dem Arbeitshub der Mittel 20, 30 entspricht.

Wie auch die Einheit 3 bildet die Einheit 2 ein topfförmiges Gehäuse aus Stirnwand 63 und zwei mit Radialabstand ineinander liegenden Mänteln 64, 65. Sie stehen nur in Richtung 12 und von der Stirnwand 63 ab. Das freie Ende des Innenmantels 65 bildet einen Kolben 66 bzw. eine Kolbenlippe. Sie gleitet am Innenumfang des Mantels 51 bis zur Stirnwand 48. In der Achse 10 und nur in Richtung 12 steht vom Boden 63 ein Ventilgehäuse 67 einschließlich der zugehörigen Teile eines Einlaßventiles vor. Dadurch bilden die Mittel 8 eine Druckluft-Pumpe 50, nämlich eine Schubkolbenpumpe. Sie saugt beim Rückhub durch das Überdruck-Kugelventil 67 von außen Luft nach. Die Außenseite der Wand 63 bildet die andere Handhabe 69, welche bei der Betätigung gemeinsam mit der Handhabe 28 mit einer Hand zu greifen ist.

Steuer- bzw. Öffnungsmittel 70 sind vorgesehen, um unabhängig vom Hubweg der Mittel 20, 30, 60 nach einem bestimmten Pumphub das Ventil 9 zu öffnen. Das Ventil 9 kann auch ab einem bestimmten Überdruck in der Pumpenkammer öffnen. Die Mittel 70 weisen in der Achse des Gliedes 55 einen Dorn 71 auf, welcher in Richtung 12 von den Teilen 63, 67 frei vorsteht. Der Dorn 71 taucht gegen Ende des Pumphubes durch die Einlaßöffnung des Gehäuses 53 ein, schlägt dann an der Kugel 55 an und nimmt sie über einen kleinen Öffnungshub mit. Das Öffnungsglied 71 hat eine Kappe, mit der es auf das freie Ende des Gehäuses 67 aufgeschnappt ist und dieses Ende verschließt.

Die Einheiten 2, 3 sind mit einer Abzugsicherung und Schnappverbindung 73 dagegen formschlüssig gesichert, voneinander abgezogen zu werden. Die Einheiten sind mit einer Drehsicherung 74 dagegen formschlüssig gesichert, relativ zueinander um die Achse 10 verdreht zu werden. Auch die Einheiten 2, 4 sind gegen Abziehen voneinander durch eine Schnappsicherung 75 gesichert. Zu diesem Zweck weist der Mantel 64 am freien Ende einen verdickten Randwulst 76 auf. Der geht über den Innenumfang des Mantels 64 unterbrochen durch und bildet vorstehende Schnapp- und Drehsicherungsglieder der Sicherungen 73, 74. Diese Glieder haben entlang des Außenumfangs des Mantels 64 Zwischenabstände. Für diese Glieder weist der Innenumfang des Mantels 37 nutförmige Sicherungsvertiefungen 74 auf. So ist am freien Ende des Mantels 37 ein Schnappglied 77 der Sicherung 73 gebildet, das zwischen den Seitenflanken der jeweiligen Nut radial nach innen vorsteht. Der Mantel 58 weist am freien Ende ein Schnappglied 78 der Sicherung 75 auf, das über seinen Außenumfang vorsteht und über seinen Umfang durchgehend bzw. abgedichtet am Innenumfang des Mantels 64 gleiten kann. Das Glied 76 bildet somit Gegenglieder für drei Sicherungen 73 bis 75.

Zwischen den Umfängen der Mäntel 51, 58 ist eine schmale Ringnut gebildet. Beide Mäntel können permanent radial vorgespannt an ihren Gegenflächen 66, 64 anliegen. Das gilt auch für die Anlage des Mantels 37 am Mantel 64. Die Mäntel 51, 58 tauchen beim Pumphub in die Ringkammer 68 ein, die von den Mänteln 64, 65 flankiert und am Boden von Querrippen begrenzt ist, welche als Anschlag für das Ende des Pumphubes dienen.

Die Teile 63 bis 67 und 69 der Einheit 2 sind einteilig miteinander ausgebildet. Gleiches gilt für die Teile 36 bis 39, 77 der Einheit 3 und für die Teile 48, 49, 51 bis 54, 57, 58, 78 der Einheit 4. Im Falle der Einheit 3 könnten außer den Teilen 22 bis 26, 28, 29 auch die Teile 33, 40 zur einteiligen Ausbildung gehören.

Bei der Betätigung wird zuerst der Öffnungshub, der Stellhub und der Leerhub der Mittel 20, 30, 60 gemeinsam durchgeführt. Bevor die Drehbewegung der Mittel 30 beendet ist, durchstößt die Spitze 25 bereits axial den Verschluß 16. So bewirkt die noch weiter bis zur Achse 11 laufende Drehbewegung eine Querbewegung der Spitze 25 relativ zum Verschluß 16. Das verbessert das Aufreißen. Die Spitze 25 hat am Ende dieses Hubes den Boden der Kammer 14 noch nicht ganz erreicht. Sie liegt aber mit ihrer Einlaßöffnung vollständig innerhalb der Kammer 14. Nun wird über die Mittel 60 auch die Einheit 4 gleichlaufend mit der Einheit 3 relativ zur Einheit 2 mitgenommen. So wird die Luft in der Pumpenkammer bei geschlossenem Ventil 9 vorkomprimiert. Nach dem größten Teil 82 des Pumphubes 81 nimmt der Dorn 71 die Kugel 55 gleichlaufend in Öffnungsstellung mit. Dadurch entweicht, wie beschrieben, die komprimierte Luft ventilfrei durch den Kanal 56 bis zur Öffnung 7. Das Glied 76 überläuft dabei die Flächen 61, 62 und liegt am Hubende in der Ringnut, die von den Teilen 36 bis 38 begrenzt ist. Nach Freigabe der Handhabe 28, 69 kehren die Mittel 9, 20, 30, 40, 50, 60, 70 gleichzeitig oder unter der Kraft der Federn 33, 56, 59 in ihre Ausgangsstellungen zurück. Dabei muß die Einheit 4 relativ zur Einheit 3 axial nicht unmittelbar anschlagbegrenzt sein. Bei der Rückstellbewegung öffnet das Überdruckventil 67, so daß die Luft angesaugt wird. Bei der Rückstellbewegung braucht der Speicher 5 keine Drehbewegung auszuführen.

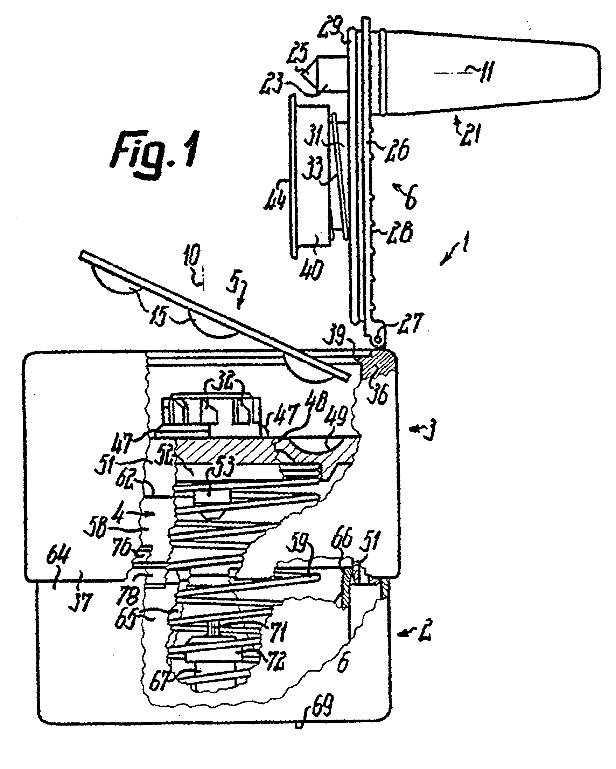

Sind alle Portionen aus dem Speicher ausgetragen, kann die Einheit 6 gemäß Figur 1 aufgeklappt werden. Hierbei laufen die Teile 21, 25, 29, 31, 36, 40 mit und machen die Teile 5, 32, 48, 49 von oben frei zugänglich. Dadurch kann der Speicher 5 gemäß Figur 1 nach oben entnommen bzw. in Richtung 13 eingesetzt werden. Alle genannten Bauteile können aus Kunststoff bzw. Spritzguß bestehen.

Alle Eigenschaften und Wirkungen können genau oder im wesentlichen bzw. nur ungefähr wie angegeben vorgesehen sein und je nach den Erfordernissen auch stärker davon abweichen. Der Abstand zwischen den Handhaben 28, 69 kann gleich dem größten Außendurchmesser des Spenders 1 sein. Jedes dieser Maße beträgt zweckmäßig höchstens 70 oder 60 mm, wobei die gezeigten Maßverhältnisse besonders günstig sind. Die Einheiten 3 bis 6 sind besonders gut zu montieren. Dabei übergreift die Einheit 3 die Einheiten 2, 4 und die Einheit 2 übergreift die Einheit 4 jeweils als Deckel am Außenumfang.

## Patentansprüche

1. Spender für Medien, insbesondere Pulver, der zwei aus einer Ruhe- in eine Endstellung in einer Arbeitsbewegung gegeneinander bewegbare, jeweils einen Grundkörper (63 bis 65 bzw. 36 bis 38) umfassende Basis-Einheiten (2, 3) umfaßt, die mit einem Medien-Speicher (5), wie einem Portionen-Speicher mit mindestens einer an einer Speicheröffnung (17) hermetisch versiegelten Speicherkammer (14), verbindbar sind und einen Medien-Auslaß (7) sowie einen Auslaß-Verschluß (9, 16) umfassen, wobei Mittel (8, 60) zur Bewegung des Speichers (5) gegenüber beiden Einheiten (2, 3) mit der Arbeitsbewegung vorgesehen sind, **dadurch gekennzeichnet, daß** der Spender einen Speicher (5) beinhaltet, welcher eine über den Außenumfang der Speicherkammer (14) vorstehende Speicherplatte (18) mit durchgehend etwa konstanter Dicke umfaßt, und daß die Speicherkammer (14) den am weitesten über die Speicherplatte (18) quer vorstehenden Teil des Speichers (5) bildet, und daß die Speicheröffnung im Axialschnitt schräg flankiert ist.

2. Spender nach Anspruch 1 **dadurch gekennzeichnet, daß** der Speicher (5) an einem Speicherträger (4) auswechselbar angeordnet ist, der gegenüber beiden Einheiten (2, 3) in einer oder zwei zueinander querliegenden Bewegungsrichtungen bewegbar gelagert ist, und daß der Speicher (5) eine Medienkapsel aus einem Kapselnapf (15) und einer dessen Speicheröffnung (17) als zerstörbarer Verschluß versiegelnden Napf-Abdeckung (16) umfaßt.

3. Spender nach Anspruch 1 oder 2, **dadurch gekennzeichnet, daß** der Speicher (5) in Richtung (13) der Arbeitsbewegung gegenüber beiden Einheiten (2, 3) bewegbar ist.

4. Spender nach Anspruch 3, **dadurch gekennzeichnet, daß** der Speicher (5) nur über einen Teilweg (81) der Arbeitsbewegung gegenüber beiden Einheiten (2, 3) und über einen anderen Teilweg (80) nur gegenüber einer Einheit (3) bewegbar ist.

5. Spender nach einem der vorhergehenden Ansprüche, **dadurch gekennzeichnet, daß** Mittel (20) zur nur einseitigen Öffnung der Speicherkammer (14) auf einem Teilweg (80) der Arbeitsbewegung auf einem anderen Teilweg (81) vorgesehen sind.

6. Spender nach einem der vorhergehenden Ansprüche, **dadurch gekennzeichnet, daß** für ein Zweitmedium eine Druckkammer, wie eine von zwei gegeneinander bewegbaren Begrenzern, nämlich einen Zylinder (51) und einem Verdränger (66), begrenzte Pumpkammer einer Pumpe (50) vorgesehen ist, daß ein Speicher (5) mit dem Zylinder (51) bzw. dem Auslaß (7) gegenüber der ersten Einheit (2) bei der Arbeitsbewegung bewegbar ist.

7. Spender nach einem der vorhergehenden Ansprüche, **dadurch gekennzeichnet, daß** eine mechanische Zwangssteuerung (70) zur Öffnung des Verschlusses (9) für ein Zweitmedium vorgesehen ist, daß die erste Einheit (2) einen Stößel (71) zur Öffnung des Verschlusses (9) kurz vor Erreichen der Endstellung umfaßt, und daß der Verschluß (9) ein reversibel arbeitendes Überdruckventil mit einem hin und her bewegbaren Ventilkörper (55) ist.

8. Spender nach einem der vorhergehenden Ansprüche, **dadurch gekennzeichnet, daß** Rückstell-Mittel, wie mindestens eine Feder (33, 59), zur Rückstellung der Einheiten (3 bis 4) aus der Endstellung in die Ruhestellung vorgesehen sind, die unmittelbar auf den Speicher (5) wirken.

9. Spender nach einem der vorhergehenden Ansprüche, **dadurch gekennzeichnet, daß** Stellmittel (30) zur Ausrichtung einer Speicherkammer (14) aus einer Ausgangsstellung in eine Bereitschaftsstellung für die unmittelbare Strömungs-Entleerung der Speicherkammer (14) zum Medienauslaß (7) hin vorgesehen sind, die einen die Arbeitsbewegung in eine Querbewegung des Speichers (5) umsetzenden Stelltrieb (31, 32) umfassen

10. Spender nach Anspruch 9, **dadurch gekennzeichnet, dass** die Stellmittel (30) derart zentral angeordnet sind, dass der Innenumfang der Speicherplatte (18) in Drehrichtung darin eingreift und zur Querbewegung drehend mitgenommen wird.

11. Spender nach einem der vorhergehenden Ansprüche, **dadurch gekennzeichnet, daß** der Speicher (5) eine Mehrzahl gesonderter Speicherkammern (14) umfaßt, die mit den Stellmitteln (30) aufeinanderfolgend in Bereitschaftsstellung überführbar sind.

12. Spender nach einem der vorhergehenden Ansprüche, **dadurch gekennzeichnet, daß** ein stromaufwärts von der Speicherkammer (14) liegender Auslaß (57) für ein Zweitmedium in einen den Speicher (5) sichernden Leitkörper (40, 41) mündet, der die Speicherkammer (14) bei der Entleerung überdeckt und ein Zweitmedium unmittelbar in die geöffnete Speicherkammer (14) leitet, und als federbelasteter Niederhalter für den Speicher (5) an der zweiten Einheit (3) bewegbar gelagert ist.

13. Spender nach einem der vorhergehenden Ansprüche, **dadurch gekennzeichnet, daß** die mit Führungsgliedern (76 bis 78) aneinander gelagerten Einheiten (2 bis 4) ein den Speicher (5) vollständig umschließendes, jedoch zum Auswechseln des Speichers (5) im Abstand von den Führungsgliedern zu öffnendes Gehäuse bilden.

14. Spender nach Anspruch 13, **dadurch gekennzeichnet, dass** ein das Gehäuse öffenbar verschließender Deckel (6) eine Stirnwand (26) einer der Einheiten (3) umfaßt den Medienauslaß (7) und/oder den Leitkörper (40, 41) umfaßt.

## Claims

1. A dispenser for media, more particularly powder, including mutually movable from a resting position into an end position in a working movement and each comprising a base body (63 to 65 or 36 to 38) two base units (2, 3) for connecting to a medium reservoir (5) such as a dosage reservoir including at least one reservoir chamber (14) hermetically sealed at a reservoir opening (17), and a medium orifice (7) as well as an outlet closure (9, 16), means (8, 60) being provided for moving said reservoir (5) relative to both units (2, 3) in said working movement, **characterized in that** said dispenser includes a reservoir (5) comprising a reservoir plate (18) protruding beyond the outer circumference of said reservoir chamber (14) roughly constant in thickness throughout and said reservoir chamber (14) forms the portion of said reservoir (5) transversely protruding the most beyond said reservoir plate (18) and said reservoir opening (17) being flanked inclined in axial section.

2. The dispenser as set forth in claim 1, **characterized in that** said reservoir (5) is interchangeably arranged on a reservoir support (4) and mounted movable relative to both units (2, 3) in one or two movement directions located transversely to each other, and said reservoir (5) comprising a medium capsule of a capsule dish (15) and dish lid (16) sealing off said reservoir opening (17) thereof as a destructible closure.

3. The dispenser as set forth in claim 1 or 2, **characterized in that** said reservoir (5) is movable in the direction (13) of said working movement relative to said two units (2, 3).

4. The dispenser as set forth in claim 3, **characterized in that** said said reservoir (5) is movable only over a travel portion (81) of said working movement relative to both units (2, 3) and over another travel portion (80) only relative to one unit (3).

5. The dispenser as set forth in any of the preceding claims, **characterized in that** means (20) are provided for opening said reservoir chamber (14) on one side only on a travel portion (80) of said working movement on another travel portion (81).

6. The dispenser as set forth in any of the preceding claims, **characterized in that** for a second medium a pressure chamber such as a pumping chamber of a pump (50) defined by two mutual movable definitions, namely a barrel (51) and an impeller (66) is provided, a reservoir (5) being movable with said barrel (51) or said orifice (7) relative to said first unit (2) in said working movement.

7. The dispenser as set forth in any of the preceding claims, **characterized in that** a mechanical control (70) for opening said closure (9) for a second medium is provided, said first unit (2) comprising a finger (71) for opening said closure (9) shortly before attaining said end position, and said closure (9) being a reversible working pressure-relief valve including a recipocatingly movable valve element (55).

8. The dispenser as set forth in any of the preceding claims, **characterized in that** return means, such as at least one spring (33, 59), are provided for returning said units (3, 4) from said end position into said resting position and acting directly on said reservoir (5).

9. The dispenser as set forth in any of the preceding claims, **characterized in that** positioning means (30) for orienting said reservoir chamber (14) from a starting position into a standby position are provided for directly flow-emptying said reservoir chamber (14) to said medium orifice (7) and comprising a positioning drive (31, 32) translating said working movement into a transverse movement of said reservoir (5).

10. The dispenser as set forth in claim 9, **characterized in that** said positioning means (30) are arranged so centrally that the inner circumference of said reservoir plate (18) engages therein in the direction of rotation and is rotatingly slaved to said transverse movement.

11. The dispenser as set forth in any of the preceding claims, **characterized in that** said reservoir (5) comprises a plurality of separate reservoir chambers (14) translatable by said positioning means (30) in sequence into said standby position.

12. The dispenser as set forth in any of the preceding claims, **characterized in that** an outlet (57) for a second medium located upstream of said reservoir chamber (14) ports into a guide body (40, 41) locking said reservoir (5), said guide body (40, 41) covering said reservoir chamber (14) during emptying and guiding a second medium directly into said opened reservoir chamber (14) and said guide body (40, 41) being movably mounted on said second unit (3) as a spring-loaded holddown for said reservoir (5).

13. The dispenser as set forth in any of the preceding claims, **characterized in that** said units (2 to 4) mounted juxtaposed with said guide means (76 to 78) form a housing totally surrounding said reservoir (5) but requiring opening for replacing said reservoir (5) spaced away from said guide means.

14. The dispenser as set forth in claim 13, **characterized in that** an openable lid (6) closing off said housing comprises a face wall (26) of one of said units (3), said medium orifice (7) and/or said guide body (40, 41).

## Revendications

1. Distributeur de substances, notamment en poudre, qui comprend deux unités de base (2, 3) qui peuvent être réciproquement déplacées par un mouvement de travail d'une position de repos en une position terminale et qui comportent respectivement un corps de base (de 63 à 65 ou encore de 36 à 38), les unités de base pouvant être raccordées à un réservoir à substance (5), tel qu'un réservoir à portions avec au moins une chambre de stockage (14) hermétiquement scellée à une ouverture de réservoir (17) et comprenant une sortie (7) pour substances ainsi qu'une fermeture d'échappement (9, 16), des moyens (8, 60) étant prévus pour le déplacement du réservoir (5) par rapport aux deux unités (2, 3) avec le mouvement de travail, **caractérisé en ce que** le distributeur possède un réservoir (5), qui comprend une plaque de réservoir (18) qui saille au-delà du périmètre extérieur de la chambre de stockage (14) et qui présente une épaisseur de bout en bout à peu près constante et **en ce que** la chambre de stockage (14) forme la partie de réservoir (5) qui saille transversalement le plus au-delà de la plaque de stockage (18), et **en ce qu'**en section axiale l'ouverture de réservoir est flanquée obliquement.

2. Distributeur d'après la revendication 1, **caractérisé en ce que** le réservoir (5) est disposé de manière à pouvoir être remplacé auprès d'un support de réservoir (4), qui est logé de manière déplaçable par rapport aux deux unités (2, 3) dans une ou deux directions de mouvement transversales l'une par rapport à l'autre, et **en ce que** le réservoir (5) comprend une capsule à substances composée d'une cuvette de capsule (15) et d'un recouvrement de cuvette (16) scellant l'ouverture de réservoir (17) de celle-ci en tant que fermeture destructible.

3. Distributeur d'après la revendication 1 ou 2, **caractérisé en ce que** le réservoir (5) est déplaçable par rapport aux unités (2, 3) en direction (13) du mouvement de travail.

4. Distributeur d'après la revendication 3, **caractérisé en ce que** le réservoir (5) est déplaçable par rapport aux deux unités (2, 3) uniquement sur une voie partielle (81) du mouvement de travail et uniquement déplaçable par rapport à l'unité (3) sur une autre voie partielle (80).

5. Distributeur d'après une des revendications précédentes, **caractérisé en ce que** des moyens (20) pour l'ouverture seulement unilatérale de la chambre de stockage (14) sur une voie partielle (80) du mouvement de travail sont prévus sur une autre voie partielle (81).

6. Distributeur d'après une des revendications précédentes, **caractérisé en ce que** pour une substance secondaire on prévoit une chambre sous pression, telle qu'une chambre de pompage d'une pompe (50), la chambre de pompage étant délimitée par deux limiteurs réciproquement déplaçables, c'est-à-dire par un cylindre (51) et par un déplaceur (66), **en ce qu'**un réservoir (5) avec le cylindre (51) ou encore avec la sortie (7) est déplaçable par rapport à la première unité (2) pendant le mouvement de travail.

7. Distributeur d'après une des revendications précédentes, **caractérisé en ce qu'**on prévoit pour une substance secondaire une commande forcée (70) pour l'ouverture de la fermeture (9), **en ce que** la première unité (2) comprend une tige de commande (71) pour l'ouverture de la fermeture (9) peut avant l'atteinte de la position terminale, et **en ce que** la fermeture (9) est une soupape de surpression travaillant de manière réversible avec un corps de soupape (55) pouvant être déplacé par un mouvement de va-et-vient.

8. Distributeur d'après une des revendications précédentes, **caractérisé en ce que** pour un rappel des unités (de 3 à 4) de la position terminale en position de repos on prévoit des moyens de rappel, tel qu'au moins un ressort (33, 59), agissant directement sur le réservoir (5).

9. Distributeur d'après une des revendications précédentes, **caractérisé en ce qu'**on prévoit des moyens de commande (30) pour l'alignement d'une chambre de stockage (14) à partir d'une position initiale en une position d'attente en vue du vidage par écoulement direct de la chambre de stockage (14) en direction de la sortie (7) pour substances, les moyens de commande comprenant un actionnement de commande (31, 32) transformant le mouvement de travail en un mouvement transversal du réservoir (5).

10. Distributeur d'après la revendication 9, **caractérisé en ce que** les moyens de commande (30) sont disposés de manière centrée, de sorte que le périmètre intérieur de la plaque de réservoir (18) s'y engage en direction du mouvement rotatoire et qu'il soit entraîné par rapport au mouvement transversal dans le sens de rotation.

11. Distributeur d'après une des revendications précédentes, **caractérisé en ce que** le réservoir (5) comprend un certain nombre de chambres de stockage (14), qui peuvent être transportées consécutivement en position d'attente par les moyens de commande (30).

12. Distributeur d'après une des revendications précédentes, **caractérisé en ce qu'**une sortie (57), en amont de la chambre de stockage (14), pour une substance secondaire débouche dans un corps de guidage (40, 41) qui assure le réservoir (5) et qui recouvre la chambre de stockage (14) pendant le vidage, et qui guide une substance secondaire directement dans la chambre de stockage (14) ouverte, et qui est logé de manière déplaçable sur la deuxième unité (3) en tant qu'abaisseur chargé par ressort pour le réservoir (5).

13. Distributeur d'après une des revendications précédentes, **caractérisé en ce que** les unités (de 2 à 4) logées l'une sur l'autre par des éléments de guidage (de 76 à 78) forment un boîtier qui enferme entièrement le réservoir (5) mais qui peut être ouvert à une certaine distance des éléments de guidage pour remplacer le réservoir (5).

14. Distributeur d'après la revendication 13, **caractérisé en ce qu'**un couvercle (6) fermant le boîtier de manière à pouvoir être ouvert, qui comprend une paroi frontale (26) d'une des unités (3), comprend la sortie (7) pour substances et/ou le corps de guidage (40, 41).
